# DEMANDE DE BREVET EUROPEEN

(11) **EP 1 614 444 A1**
(43) Date de publication de la demande: **11.01.2006**
(21) Numéro de dépôt: 05106103.4
(22) Date de dépôt: 05.07.2005
(51) Int. Cl.: A61N 1/378

(54) **Stimulateur cardiaque multi-sites à réseau d'électrodes asservies**

(30) Priorité: 07.07.2004 FR 0407567
(71) Demandeur: COMMISSARIAT A L'ENERGIE ATOMIQUE, 75752 Paris Cedex 15 (FR)
(72) Inventeur: Cohen, Josy, 92290 Chatenay-Malabry (FR); Joffre, Francis, 91440 Bures sur Yvette (FR); Salichon, Maurice, 91190, Gif sur Yvette (FR); Bonnet, Nicolas, 75003 Paris (FR); Karouia, Mourad, 92220 Bagneux (FR)
(74) Mandataire: Poulin, Gérard

(57) **Abrégé**

L'invention concerne un stimulateur cardiaque implantable. Ce stimulateur cardiaque comprend au moins un câble conducteur (11) reliant électriquement un boîtier de commande (10) à au moins une électrode placée en un point du coeur d'un patient, ledit boîtier de commande (10) comprenant un système d'alimentation et un système électronique, et est caractérisé en ce qu'au moins une partie dudit système électronique est délocalisée du boîtier de commande (10) et est située au niveau de la au moins une électrode pour former un module d'électrode (13,14a,14b), le module d'électrode étant placé sur la surface extérieure du coeur.

## Description

### DOMAINE TECHNIQUE

L'invention concerne un stimulateur cardiaque permettant de stimuler et/ou de sonder le coeur en péricardique (sur sa face extérieure), ou en endocavitaire (à l'intérieur des cavités cardiaques), à l'aide d'électrodes en des points judicieusement choisis du coeur. En particulier, l'invention concerne un stimulateur cardiaque multi-sites comprenant des électrodes asservies disposées sur les mailles d'un réseau et dans lequel chaque site est équipé d'une électrode pour stimuler et/ou sonder le coeur.

Ce dispositif est destiné en particulier aux patients présentant des insuffisances cardiaques.

### ÉTAT DE LA TECHNIQUE ANTÉRIEURE

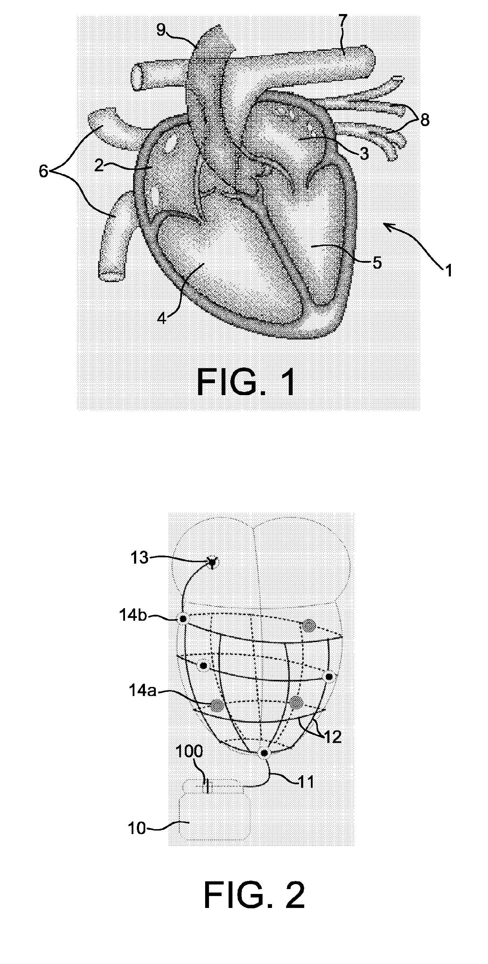

Le coeur est un organe constitué de fibres ou cellules musculaires qui ont la particularité de pouvoir propager des signaux électriques qui, en se propageant, provoquent la contraction des oreillettes et des ventricules du coeur. Le signal électrique est créé en un point précis du coeur, le noeud sinusal, situé dans l'oreillette droite. Le noeud sinusal émet régulièrement des impulsions électriques faibles qui sont transmises par un relais, le noeud auriculo-ventriculaire, aux fibres musculaires cardiaques. Pour obtenir une contraction, les cellules musculaires cardiaques se dépolarisent électriquement, créant ainsi une onde de dépolarisation qui, de proche en proche, provoque la dépolarisation électrique de toutes les cellules musculaires du coeur. Après le passage de cette onde et au bout d'un temps déterminé, une onde de contraction mécanique se propage dans les oreillettes et les ventricules, contribuant ainsi au pompage du sang dans et hors du coeur. Sur la figure 1, on peut voir un schéma d'un coeur 1 comprenant une oreillette droite 2 et une oreillette gauche 3 dans la partie supérieure du coeur, un ventricule droit 4 et un ventricule gauche 5 dans la partie inférieure. Le sang arrive dans l'oreillette droite 2 par les veines caves 6, passe dans le ventricule droit 4 puis va dans les poumons par les artères pulmonaires 7. Le sang revient ensuite par les veines pulmonaires 8 et entre dans l'oreillette gauche 3, puis dans le ventricule gauche 5 et ressort par l'aorte 9. Quand le coeur a un fonctionnement défaillant, on peut le stimuler en créant en un point donné du coeur une nouvelle onde de dépolarisation électrique ou en relançant une onde qui n'a pas abouti. Pour réaliser cette stimulation cardiaque, on utilise généralement un appareil appelé stimulateur cardiaque ou « pacemaker » en anglais, qui est implanté dans le corps du patient, près du coeur.

Actuellement, les stimulateurs cardiaques implantables sont constitués d'un boîtier de commande, d'au moins un câble conducteur et d'au moins une électrode. Le boîtier de commande comprend un système d'alimentation (une batterie ou une pile) capable de délivrer des impulsions électriques à intervalles réguliers et une électronique de commande plus ou moins complexe qui permet de contrôler les intervalles entre les impulsions électriques délivrées. Ces impulsions électriques sont transmises jusqu'au coeur par le câble conducteur, appelé câble d'électrode, qui établit le contact électrique entre le boîtier et le coeur. A l'extrémité du câble est disposé une électrode qui est mise en contact avec le coeur. A l'extrémité d'un même câble, on peut disposer plusieurs électrodes. De même, le stimulateur cardiaque peut disposer de plusieurs câbles. Parmi les électrodes, certaines peuvent avoir une fonction de stimulation, et d'autres une fonction de sonde de l'état électrique du coeur. Elles peuvent aussi assurer ces deux fonctions simultanément.

Le problème des stimulateurs cardiaques actuels est qu'il est nécessaire de changer leur boîtier de commande environ tous les 4 ans, à cause de l'usure du système d'alimentation. Or, chaque changement nécessite d'effectuer une nouvelle opération du patient, avec tous les risques que cela comporte, comme par exemple les risques d'infection, de maladies nosocomiales, ou les risques liés à l'intervention chirurgicale en elle-même.

Par ailleurs, les électrodes des stimulateurs cardiaques actuels doivent être positionnées individuellement sur le coeur. Le praticien doit les positionner une à une en les insérant dans le coeur en endocavitaire à travers des veines, constater leurs effets et, éventuellement, les repositionner à nouveau afin de trouver un fonctionnement du coeur amélioré et optimisé. Le positionnement des électrodes sur le coeur est donc une étape difficile et fastidieuse.

Un autre problème réside dans l'encombrement des câbles d'électrodes. En effet, les câbles d'électrodes et les électrodes sont dans la plupart des cas acheminées jusqu'au coeur en étant introduits dans des veines ou des artères conduisant au coeur (voie endocavitaire). Un exemple d'un tel stimulateur cardiaque peut être étudié dans le document **[1]**. Dans un tel stimulateur cardiaque, le nombre des câbles d'électrodes est limité par le diamètre des veines ou des artères, ainsi que par la taille des câbles d'électrodes. Actuellement, nous trouvons sur le marché des stimulateurs cardiaques comportant un maximum de 3 câbles d'électrodes. On comprend aisément que la multiplication des sites de stimulation avec des stimulateurs cardiaques actuels aboutira à une limite technologique constituée par une impossibilité anatomophysiologique, ne serait-ce que par l'encombrement stérique des câbles d'électrodes.

Pour résoudre ce problème, il est possible de réaliser des câbles d'électrodes comportant des ramifications. Des exemples de stimulateurs cardiaques présentant de tels câbles d'électrodes ramifiés peuvent être étudiés dans les documents **[1]** et **[2]**. L'inconvénient de cette solution est que la ramification des câbles d'électrodes entraîne une augmentation de la résistance électrique dans les câbles et provoque une chute de la tension. Or, pour propager une onde de dépolarisation électrique dans les cellules musculaires cardiaques, il est nécessaire d'initier une stimulation suffisante sur un point du coeur selon certaines conditions électriques minimales (en tension, et en puissance ou en énergie). Avec ce système, la puissance électrique nécessaire pour stimuler de manière homogène chaque site de stimulation ne peut pas être fournie par la pile habituellement utilisée dans la technologie des stimulateurs cardiaques. L'utilisation de câbles d'électrodes ramifiés nécessite de disposer d'un système d'alimentation plus important en capacité et en tension délivrée, et par conséquent, d'un système plus volumineux et plus lourd.

### EXPOSÉ DE L'INVENTION

Le but de l'invention est de fournir un stimulateur cardiaque destiné à être implanté dans le corps d'un patient et ne présentant pas les inconvénients de l'art antérieur. Ce but est atteint grâce à un stimulateur cardiaque implantable comprenant au moins un câble conducteur reliant électriquement un boîtier de commande à au moins une électrode placée en un point du coeur d'un patient, le boîtier de commande comprenant un système d'alimentation et un système électronique, caractérisé en ce qu'au moins une partie du système électronique est délocalisée du boîtier de commande et est située au niveau de la au moins une électrode pour former un module d'électrode, le module d'électrode étant placé à la surface du coeur.

Selon une variante, au moins une partie du système d'alimentation est délocalisée du boîtier de commande et est située au niveau d'au moins un module d'électrode.

Avantageusement, la partie du système électronique délocalisée est une électronique de traitement et/ou de commande, cette électronique permettant au module de sonder et/ou de stimuler le coeur.

Les modules d'électrodes sont donc constitués d'une électrode et d'une électronique de traitement et/ou de commande. Ces modules d'électrodes sont positionnés sur le coeur en des sites judicieusement choisis.

L'électronique de traitement permet le traitement d'un signal, tandis que l'électronique de commande permet ici d'exercer une commande sur le déclenchement d'une stimulation.

Si le module d'électrode ne comporte qu'une électronique de traitement, alors il ne peut fonctionner qu'en mode sonde, c'est-à-dire qu'il étudie le comportement du coeur.

Si le module d'électrode ne comporte qu'une électronique de commande, alors il ne peut fonctionner qu'en mode de stimulation.

Si le stimulateur cardiaque ne dispose que de module d'électrode en mode de stimulation, alors les stimulations du coeur ont lieu à intervalles réguliers, quel que soit l'état du coeur, c'est-à-dire que le coeur ait besoin ou non d'une stimulation artificielle.

Dans le cas où le stimulateur cardiaque comporte à la fois des modules d'électrodes en mode sonde et en mode de stimulation, il est intéressant de réaliser un asservissement des modules d'électrodes en mode de stimulation sur les modules d'électrodes en mode sonde. En d'autres termes, on fait en sorte que les modules d'électrodes en mode de stimulation soient dépendants des informations fournies par les modules d'électrodes en mode sonde. On dispose ainsi d'un moyen de régulation du rythme de la stimulation réalisée par les modules en mode de stimulation sur le rythme cardiaque nécessaire pour pallier à l'insuffisance cardiaque du patient équipé du stimulateur. Ainsi, le stimulateur cardiaque n'envoie des stimulations que lorsque le coeur en a besoin. Le stimulateur s'adapte au rythme de vie du patient.

Enfin, chaque module d'électrode peut comporter à la fois une électronique de traitement et une électronique de commande. Dans ce cas, chaque module d'électrode peut commuter et être configuré en mode sonde ou stimulation selon le besoin. Dans ce cas, le module d'électrode peut traiter tous signaux provenant du coeur, traiter tous signaux provenant des autres modules et exercer une commande sur le déclenchement d'une stimulation. En général, le module d'électrode sera en mode sonde la plupart du temps et passera en mode de stimulation que si il perçoit le besoin d'une stimulation du coeur.

Avantageusement, l'électronique de traitement permettant au module d'électrode de sonder le coeur comprend au moins un capteur. Ce capteur peut être choisi parmi les capteurs suivants :
- un ou des accéléromètres permettant d'adapter le rythme cardiaque au rythme de vie du patient équipé,
- un ou des accéléromètres permettant de mesurer la contractilité du myocarde (contraction des ventricules et des oreillettes équipés),
- un ou des capteurs de respiration (mouvement de la cage thoracique) qui permet avec un accéléromètre de bien mieux adapter le rythme cardiaque au rythme de vie du patient,
- un ou des capteurs de mouvement permettant de réaliser les mêmes fonctionnalités que l'accéléromètre,
- un ou des capteurs piézo-électriques permettant de capter l'activité du coeur,
- un ou des capteurs de température du sang,
- un ou des capteurs d'oxygénation du sang,
- un ou des capteurs de pression sanguine,
- un ou des débitmètres pour mesurer le débit sanguin,
- une ou des sondes permettant de capter l'onde de dépolarisation du myocarde...

Avantageusement, l'électronique de traitement permettant au module d'électrode de sonder le coeur comprend un moyen apte à sélectionner des signaux de fréquence déterminée. Avantageusement, ce moyen est un filtre.

L'électronique de traitement, et en particulier le filtre, est placée soit dans le module d'électrode, soit dans le boîtier de commande, selon qu'on ait une délocalisation partielle ou totale du système électronique.

Avantageusement, l'électronique de commande permettant au module d'électrode de stimuler le coeur comprend un moyen apte à délivrer une stimulation électrique au coeur. Avantageusement, ce moyen est un condensateur.

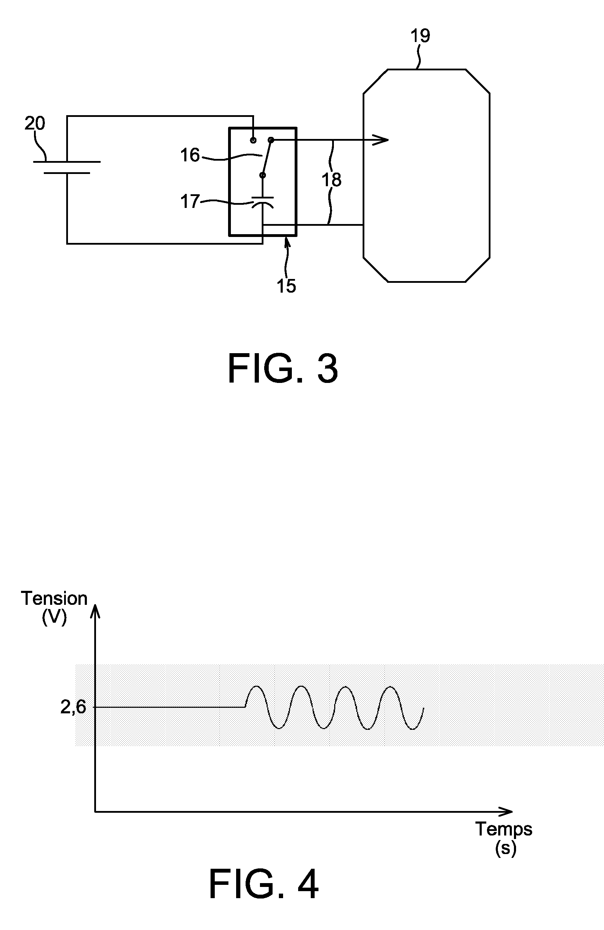

Dans l'exemple de la figure 3, le condensateur est à l'intérieur de l'électronique de traitement. Le condensateur peut être dans le module d'électrode ou être dans le boîtier de commande selon le niveau de délocalisation.

Avantageusement, le système électronique localisé au niveau des modules d'électrodes est miniaturisé.

Avantageusement, la partie du système électronique délocalisée est un système sur puce. La conception d'un système sur puce (ou SoC pour « system on chip » en anglais) est réalisée de préférence à l'aide de la microtechnologie. On utilisera par exemple les derniers développements de la microtechnologie (ASIC, FPGA, MENS, ...).

Selon un cas particulier, le stimulateur cardiaque comprend au moins deux modules d'électrodes reliés entre eux par un moyen qui entoure au moins une partie du coeur.

Ce moyen est placé sur l'ensemble ou seulement une partie du coeur. On peut en effet n'avoir à stimuler qu'une partie particulière du coeur, par exemple dans le cas où les autres parties du coeur sont constituées de tissus sains.

Avantageusement, le moyen qui entoure tout ou partie du coeur sert au maintien mécanique des au moins deux modules d'électrodes.

Avantageusement, le moyen est un moyen servant à la contention de la partie du coeur entourée par ce moyen. C'est particulièrement utile lorsqu'on doit traiter un patient souffrant d'hypertrophie ventriculaire.

Avantageusement, le moyen est un moyen servant à la communication électrique entre les modules d'électrodes.

Avantageusement, le moyen est un moyen servant à la distribution d'énergie entre les modules d'électrodes.

Le moyen peut ainsi servir de maintien mécanique des modules d'électrodes sur le coeur, de contention du coeur, de communication entre les modules d'électrodes et/ou de distributeur d'énergie.

Avantageusement, si le moyen a au moins un rôle de distributeur d'énergie, alors ce moyen ou un module d'électrode situé sur ce moyen peut être relié au boîtier de commande par un câble conducteur. Dans ce cas particulier, un seul câble conducteur fournit l'alimentation électrique à l'ensemble des modules d'électrodes situés sur ce moyen.

De préférence, les parties constitutives du stimulateur cardiaque en contact avec le coeur et son environnement sont en matériaux biocompatibles. De plus, les parties constitutives du stimulateur cardiaque en contact avec le coeur et son environnement, à l'exception des électrodes, sont en un matériau isolant. Par exemple, ces parties peuvent être enrobées de silicone ou de titane.

Avantageusement, le moyen permettant la communication entre les modules d'électrodes et/ou la distribution d'énergie est un câble coaxial multifilaire.

Avantageusement, le moyen permettant le maintien mécanique des modules d'électrodes sur le coeur, la contention du coeur, la communication entre les modules d'électrodes et/ou la distribution d'énergie est un filet comportant un maillage. Par exemple, il peut s'agir d'un ensemble de mailles entrecroisées. Les modules d'électrodes sont positionnés sur les mailles du filet en des sites judicieusement choisis, pouvant se situer aux noeuds (intersection) des mailles ou non. Le maillage du filet pourra être régulier ou non, dense ou non.

Les modules d'électrodes sont organisés en réseau sous forme de maille, de filet, de grappe, d'arborescence ou de tout autre géométrie adaptée à une application cardiaque permettant de stimuler le coeur au plus juste en terme d'énergie et de motif spatio-temporel de stimulation assurant la meilleure coordination de contraction de chacune des cavités cardiaques au cours d'un cycle.

Avantageusement, le maillage du filet est réalisé à partir de conducteurs hélicoïdaux multifilaires et coaxiaux, recouverts d'un matériau électriquement isolant et biocompatible avec les tissus humains avec lesquels ils sont en contact. Cela est particulièrement pratique lorsque les modules d'électrodes comprennent à la fois une électronique de traitement et une électronique de commande. En effet, la présence de conducteurs multifilaires permet le fonctionnement des modules d'électrodes en mode bipolaire dans un circuit électrique fermé, c'est-à-dire, en mode sonde ou en mode de stimulation : l'énergie électrique est amenée aux électrodes pour stimuler ou sonder le myocarde par un circuit électrique fermé.

Selon un mode de réalisation particulier, le stimulateur cardiaque implantable selon l'invention comprend en outre un moyen de communication permettant de configurer à distance les modules d'électrodes, ledit moyen de communication étant situé dans le boîtier de commande. Le praticien pourra ainsi, sans avoir à opérer le patient pour accéder au boîtier de commande et aux modules d'électrodes, modifier le fonctionnement des modules d'électrodes, par exemple en faisant commuter un module d'électrode déterminé en mode sonde plutôt qu'en mode de stimulation.

Avantageusement, ce moyen de communication comporte une antenne et fonctionne par télétransmission.

Avantageusement, seule l'antenne du module de communication est situé dans le boîtier de commande, le reste des éléments constituant le moyen de communication étant localisé sur le moyen permettant de relier les modules d'électrodes.

Il peut s'agir de modules d'électrodes ne comportant qu'une électronique de commande, ou bien de modules d'électrodes ayant à la fois une électronique de commande et une électronique de traitement que l'on fait commuter en mode de stimulation.

Avantageusement, au moins un module d'électrode est configuré en mode sonde et transmet des informations sur l'état du coeur aux autres modules d'électrodes. Ici, le module d'électrode en question peut ne comporter qu'une électronique de traitement, ou bien comporter à la fois une électronique de commande et une électronique de traitement et être commuté en mode sonde. Un même module d'électrode peut être sonde et stimulateur.

Avantageusement, au moins un module d'électrode est configuré en mode sonde et en mode stimulation.

Avantageusement, tous les modules d'électrodes sont situés sur le moyen permettant de relier entre eux les modules d'électrodes.

Avantageusement, au moins un module d'électrode configuré en mode sonde n'est pas situé sur le moyen permettant de relier entre eux des modules d'électrodes, mais lui est relié électriquement. En fait, on peut choisir de localiser le module d'électrode configuré en mode sonde sur le moyen permettant de relier entre eux les modules d'électrodes ou bien décider de le situer en dehors dudit moyen et de le relier électriquement à lui.

Avantageusement, le module d'électrode configuré en mode sonde est un module apte à exécuter la suite d'étapes suivantes :
a)- analyser l'état électrique du coeur au niveau où le module d'électrode configuré en mode sonde est situé,
b)- à la réception d'un signal électrique provenant du coeur, calculer le niveau de fréquence dudit signal pour déterminer si le coeur est en état de dépolarisation ou en état de repolarisation,
c)- si le coeur est en état de dépolarisation, alors envoyer un signal codé en fréquence sur le moyen permettant l'échange d'information entre les modules d'électrodes, le codage en fréquence étant indicatif du temps séparant deux états de repolarisation du coeur.

Avantageusement, le stimulateur cardiaque comprend au moins un module d'électrode configuré en mode stimulation qui est apte à exécuter la suite d'étapes suivantes :
a)- attendre un signal codé en fréquence provenant d'un module en mode sonde,
b)- après la détection du signal codé, démarrer l'horloge interne et attendre pendant un temps de consigne déterminé,
c)- analyser l'état électrique du coeur au niveau où le module d'électrode en mode stimulation est situé,
d)- si il y a réception par le module d'électrode configuré en mode stimulation d'un signal électrique en provenance du coeur dont la valeur est supérieure à un seuil déterminé, alors l'horloge interne est arrêtée et le temps de consigne est remis à zéro,
e)- si aucun signal électrique provenant du coeur n'est reçu par le module d'électrode en mode stimulation, alors le module délivre une stimulation électrique au niveau du point du coeur où il est situé, l'horloge interne est arrêtée et le temps de consigne est remis à zéro.

Le module d'électrode en mode sonde surveille la fréquence des pulsations du coeur et éventuellement leur amplitude, et envoie un ordre de stimulation aux modules d'électrodes en mode de stimulation pour leur signaler qu'il faut augmenter ou diminuer le temps entre deux stimulations successives. Cela permet de réguler le rythme de la stimulation et de fournir un rythme cardiaque nécessaire pour pallier l'insuffisance cardiaque du patient équipé. Le stimulateur cardiaque s'adapte ainsi au rythme de vie du patient (marche rapide, repos, ...). De plus, grâce à la veille réalisée par le ou les modules d'électrodes en mode sonde, la stimulation n'est faite que si la zone du myocarde où est implanté le module d'électrode configuré en mode de stimulation n'est pas dans une phase de repolarisation.

### BRÈVE DESCRIPTION DES DESSINS

L'invention sera mieux comprise et d'autres avantages et particularités apparaîtront à la lecture de la description qui va suivre, donnée à titre d'exemple non limitatif, et accompagnée des dessins annexés parmi lesquels :
- la figure 1 est une vue en coupe d'un coeur humain,
- la figure 2 est une vue de face schématisée d'un coeur équipé du stimulateur cardiaque selon l'invention,
- la figure 3 représente un circuit de charge et de décharge permettant de fournir une stimulation au coeur,
- la figure 4 illustre la forme d'un signal caractéristique à fréquence donnée,
- la figure 5 représente une vue en coupe latérale d'un module d'électrode,
- la figure 6 représente une vue de dessus du module d'électrode de la figure 5,
- la figure 7 est un organigramme de fonctionnement du module d'électrode en mode sonde,
- la figure 8 est un organigramme de fonctionnement du module d'électrode en mode stimulateur,
- la figure 9 est un découpage en blocs fonctionnels de l'électronique ASIC utilisée dans les modules.

### EXPOSÉ DÉTAILLÉ DE MODES DE RÉALISATION PARTICULIERS

Pour illustrer le principe de fonctionnement du stimulateur cardiaque selon l'invention, nous allons l'appliquer au cas d'un patient atteint d'insuffisance cardiaque due à une contraction désynchronisée des ventricules.

Nous rappelons que le coeur est divisé en quatre cavités : les oreillettes gauche et droite, et les ventricules gauche et droit. La propagation de l'onde de dépolarisation part du noeud sinusal situé dans l'oreillette droite et se propage dans toute l'oreillette droite puis dans l'oreillette gauche, provoquant ainsi une contraction mécanique des oreillettes droite puis gauche qui permet d'expulser le sang dans les ventricules. Entre temps, l'onde s'est propagée vers les ventricules et provoque la contraction des ventricules gauche et droit, ce qui permet l'éjection du sang dans les artères.

Dans le cas particulier d'un patient souffrant de contractions désynchronisées des ventricules, seuls les ventricules ont besoin d'être équipées d'électrodes de stimulation. Les oreillettes peuvent toutefois comporter des électrodes, notamment des électrodes pour sonder l'état du coeur et éventuellement commander les électrodes de stimulation placées sur les ventricules. On réalise ainsi un asservissement des électrodes et on peut synchroniser les stimulations, et donc les ventricules.

Le stimulateur cardiaque implantable utilisé dans cet exemple d'application présente un boîtier de commande, un câble d'alimentation conducteur, des modules d'électrode ayant une électronique de traitement et/ou de commande, un filet à maille régulière permettant de relier entre eux les modules d'électrodes. Bien entendu, d'autres configurations sont possibles (filet à mailles non régulières, absence de filet...).

Sur la figure 2, nous avons représenté le coeur de ce patient qui, après une opération chirurgicale, se trouve équipé du dispositif de stimulation cardiaque selon l'invention. Dans cet exemple, on dispose d'un module d'électrode 13 isolé en mode sonde qui est situé sur l'oreillette droite du coeur. Ce module d'électrode 13 est relié à un boîtier de commande 10, qui peut comprendre d'autres fonctionnalités, par un câble d'alimentation 11 conducteur qui arrive au niveau du coeur en passant par une veine en endocavitaire. Le câble d'alimentation 11 est ici relié au boîtier de commande 10 par un connecteur à vis 100 permettant de fixer le câble 11 au boîtier 10. Le boîtier de commande 10 comprend un système d'alimentation et est localisé en sous-cutané, comme dans les stimulateurs cardiaques actuels. L'alimentation du stimulateur cardiaque est obtenue par une pile au lithium-iode de 2,8 V contenue dans le boîtier de commande 10. Le boîtier de commande 10 est de préférence en titane. On notera que le boîtier de commande 10 peut ne consister qu'en un système d'alimentation si toute l'électronique est délocalisée au niveau des modules d'électrodes. Le câble d'alimentation relie électriquement également un câble coaxial multifilaire 12 (représenté en pointillés sur la face non visible du coeur) qui entoure une partie des ventricules du coeur et sur lequel sont fixés des modules d'électrodes. Le câble coaxial multifilaire 12 aurait pu être remplacé par un filet par exemple à mailles régulières qui aurait entouré les ventricules et sur lequel on aurait placé les modules d'électrodes. Lors de l'opération chirurgicale, le chirurgien positionne en épicardique (c'est-à-dire à l'extérieur du coeur) le câble coaxial 12 équipé de modules d'électrode 14a (situés sur la face non visible du coeur sur la figure 2), 14b (situés sur la face visible du coeur). Dans cet exemple, on a positionné un module d'électrode 13 configuré en mode sonde au niveau de l'oreillette droite, quatre modules d'électrodes configurés en mode de stimulation au niveau du ventricule droit et encore cinq sur le ventricule gauche, les modules d'électrodes 14a, 14b situés sur les ventricules étant reliés par le câble coaxial 12. Notons que la figure 2 représente une vue de face du coeur du patient où n'apparaît qu'une partie des modules d'électrodes.

Le câble coaxial 12 (ou le filet) comprenant les modules d'électrode 14a,14b est placé sur la surface extérieure du coeur au niveau des ventricules. Les modules d'électrode 13,14a,14b représentés sur cette figure sont de forme ronde. Sur la figure 2, on voit que le câble d'alimentation 11 alimente en électricité le module d'électrode configuré en mode sonde 13 isolé et situé sur l'oreillette droite, ainsi que le câble coaxial 12 sur lequel sont situé les modules d'électrodes en mode de stimulation 14a, 14b.

Le câble coaxial 12 permet ici de maintenir les différents modules d'électrodes 14a, 14b sur le coeur, d'alimenter en électricité les modules d'électrodes qui lui sont fixés et permet aussi auxdits modules d'électrodes de communiquer entre eux. Les modules d'électrodes 13,14a,14b sont positionnés en des sites judicieusement choisis à la surface du coeur. Si le câble coaxial 12 est remplacé par un filet, les modules d'électrodes 14a, 14b reliés entre eux par les mailles du filet peuvent être ici positionnés sur les mailles en des sites judicieusement choisis, pouvant se situer aux noeuds de la maille ou non. Précisons que le filet peut recouvrir tout ou une partie seulement du coeur, et qu'il peut être à maillage régulier ou non, et dense ou non. Le filet et toutes les parties en contact avec le coeur et son environnement sont en matériaux biocompatibles (enrobage de silicone, ou titane, par exemple).

Les mailles du filet sont réalisées à partir de fils de matière conductrice. En particulier, les mailles du filet sont constituées de plusieurs fils enroulés entre eux de manière hélicoïdale et coaxiale, ces fils étant recouverts d'un matériau électriquement isolant et biocompatible avec les tissus humains. Ce matériau électriquement isolant et biocompatible peut être par exemple du silicone de haute performance. En utilisant plusieurs fils pour réaliser les mailles du filet, on peut permettre le fonctionnement des modules d'électrodes en mode bipolaire, l'énergie électrique étant amenée aux électrodes pour stimuler ou sonder le myocarde par un circuit électrique fermé.

Le filet ou le câble coaxial 12 sur lequel sont disposés des modules d'électrode 14a, 14b est assemblé avant l'opération chirurgicale. Pour faciliter son positionnement sur le coeur, le filet ou le câble coaxial équipé des modules d'électrodes nécessaires est réalisé dans des dimensions plus larges que celle du coeur. On pourra ensuite resserrer les mailles du filet après son positionnement. En resserrant plus ou moins les mailles autour du coeur, le filet 12 peut également avoir un rôle de contention du coeur (par exemple pour traiter un cas d'hypertrophie ventriculaire).

Si le filet n'a qu'un rôle de contention du coeur, les modules d'électrodes doivent être reliés à des câbles conducteurs pour recevoir de l'énergie électrique. Dans ce cas, chaque câble est équipé à ses extrémités de connecteur coaxial qui permettent une connexion facile aux modules d'électrode. La connexion pourra se faire par exemple par visserie. Dans la figure 2, le dispositif comporte un seul câble qui correspond au câble d'alimentation 11. Ici, c'est le filet qui permet d'amener l'énergie électrique aux modules d'électrodes.

Dans cet exemple d'application, le module d'électrode en mode sonde permet de conférer au stimulateur cardiaque des moyens de régulation du rythme de la stimulation réalisée par les modules d'électrodes en mode de stimulation. Dans cet exemple, on dispose bien d'un stimulateur cardiaque multi-sites à filet d'électrodes asservies, c'est-à-dire que le stimulateur comporte plusieurs sites de stimulation disposés sur un filet, les sites de stimulation étant asservis aux résultats fournis par le module d'électrode situé sur l'oreillette droite.

Les modules d'électrodes de l'invention sont constitués d'une électronique de traitement et/ou de commande, et permettent de traiter les signaux provenant du coeur et éventuellement du filet et d'exercer une commande sur le déclenchement d'une stimulation. Cette électronique de traitement et/ou de commande est constituée d'un circuit électronique spécifique (ASIC) qui permet de réaliser ce traitement et/ou cette commande. Ce circuit électronique spécifique (ASIC) est un système sur puce (SoC System on Chip), conçu à l'aide de blocs SIP (Semiconductor Intellectual Property) ou IP (Intellectual Property). Nous y trouvons une régulation d'alimentation électrique qui permet le fonctionnement de cet ASIC.

La stimulation peut être réalisée grâce à la présence d'un condensateur qui peut être actionné par un interrupteur commandé par chaque module d'électrode ayant une fonction de stimulation. La figure 3 schématise un module d'électrode pouvant fonctionner en mode de stimulation 15 qui est capable de commander l'actionnement d'un interrupteur et ainsi délivrer une stimulation grâce à la présence d'un condensateur 17 (ici, l'interrupteur et le condensateur sont placés dans le module d'électrode, mais ils peuvent aussi être délocalisés) par l'intermédiaire de l'électrode 18 (en deux parties) placée dans une zone du myocarde 19. Le condensateur 17 est rechargé grâce à une pile 20 de 2,8 V. Le condensateur 17, après chargement, permet de délivrer une impulsion suffisante pour provoquer une onde de dépolarisation par stimulation. Le chargement du condensateur peut être effectué directement par l'alimentation du boîtier de commande 10. La stimulation est ensuite déclenchée par le module d'électrode grâce à son circuit électronique (ASIC) lorsque nécessaire.

La sonde ou la captation de l'état du coeur (dépolarisation, repolarisation) est réalisée à l'aide de filtres situés dans les modules d'électrodes ayant une fonction de sonde afin de capter les signaux caractéristiques de la dépolarisation (fréquence de 10 à 120 Hz, et tension de 1 à 20 mV) et de la repolarisation (fréquence inférieure à 5 Hz et tension inférieure à 7 mV). L'ASIC permet ici en particulier de réaliser ce traitement de l'information. L'ASIC placé dans les modules d'électrodes en mode sonde permet également de réaliser l'asservissement des modules d'électrodes de stimulation en captant et en codant un signal caractéristique en tension (figure 4) afin de l'émettre vers les modules d'électrodes en mode de stimulation, qui, selon la tension reçue, stimuleront ou non le coeur. Les mailles du filet sont ici utilisées pour l'échange de ce signal entre les modules d'électrodes en mode sonde et les modules d'électrodes en mode de stimulation. Ce signal vient se superposer à la tension d'alimentation qui circule déjà dans les mailles du filet. Sur la figure 4, on peut voir un exemple de forme de signal caractéristique à fréquence donnée superposé à la tension continue d'alimentation à 2,8 V. Le signal caractéristique est codé à une fréquence donnée avec une amplitude déterminée qui est d'environ 0,25 V en général. Le signal caractéristique véhicule une information sur une fréquence, cette fréquence reflétant l'état d'activité du patient à un instant donné. En effet, pour chaque patient, nous identifions trois niveaux de rythme cardiaque : rythme de repos (inférieur à 70 pulsations/minute), rythme actif (entre 71 et 140 pulsations/minute) et rythme « super actif » (supérieur à 140 pulsations/minute). Cette information sur le rythme cardiaque est alors codée en fréquence sur le signal caractéristique, respectivement 1 kHz, 10 kHz et 100 kHz.

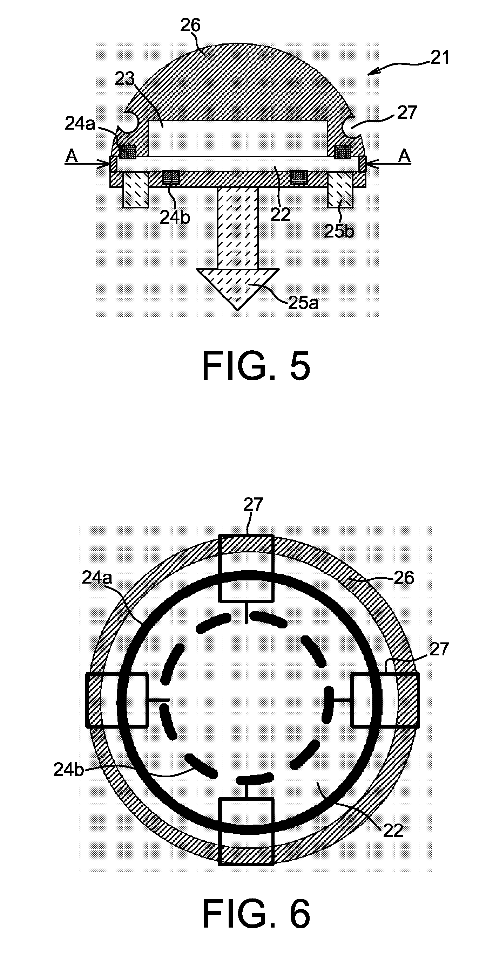

La structure des modules d'électrodes en mode sonde et/ou stimulation est illustrée dans les figures 5 et 6. Dans la figure 5, on peut voir que chaque module d'électrode 21 en mode sonde et/ou stimulation comprend un circuit imprimé 22 sur lequel est monté un circuit électronique 23 (ASIC) permettant de commander une stimulation et/ou de coder un signal. Le circuit imprimé 22 comprend sur sa face supérieure et sur sa face inférieure une piste conductrice 24a,24b permettant de relier le circuit imprimé 22 à un pôle de la pile. Ainsi, chaque piste conductrice 24a,24b est reliée à un pôle différent de la pile. Pour relier électriquement le module d'électrode 21 au coeur, on utilise une électrode 25a,25b. L'électrode 25a,25b est constituée de deux armatures en matériaux poreux, par exemple en carbone. L'une de ces armatures est située en position centrale 25a par rapport au circuit imprimé 22 et possède une l'extrémité en forme de pointe ou de flèche. Cette armature centrale 25a d'électrode peut avoir une longueur de 3 mm. L'autre armature 25b est située en périphérie du circuit imprimé et sert à fermer le circuit électrique sur la zone du coeur sur laquelle il est implanté, en vue de la sonder ou de la stimuler. Lors du positionnement du filet et du module d'électrode sur le coeur, c'est l'armature en forme de pointe 25a qui est enfoncée dans le myocarde à la position choisie. Tous les éléments constituant le module d'électrode à l'exception de l'électrode 25a,25b sont enrobés dans un matériau isolant et biocompatible avec le coeur et son environnement (par exemple du silicone). Dans l'exemple illustré dans la figure 5, le module d'électrode enrobé a une face de forme ronde et bombée sur le dessus et une face ronde et lisse sur la face opposée laissant apparaître les armatures de l'électrode 25a,25b. Dans sa partie bombée, l'enrobage en silicone 26 comporte dans cet exemple des encoches symétriques permettant la prise du module d'électrode par le chirurgien.

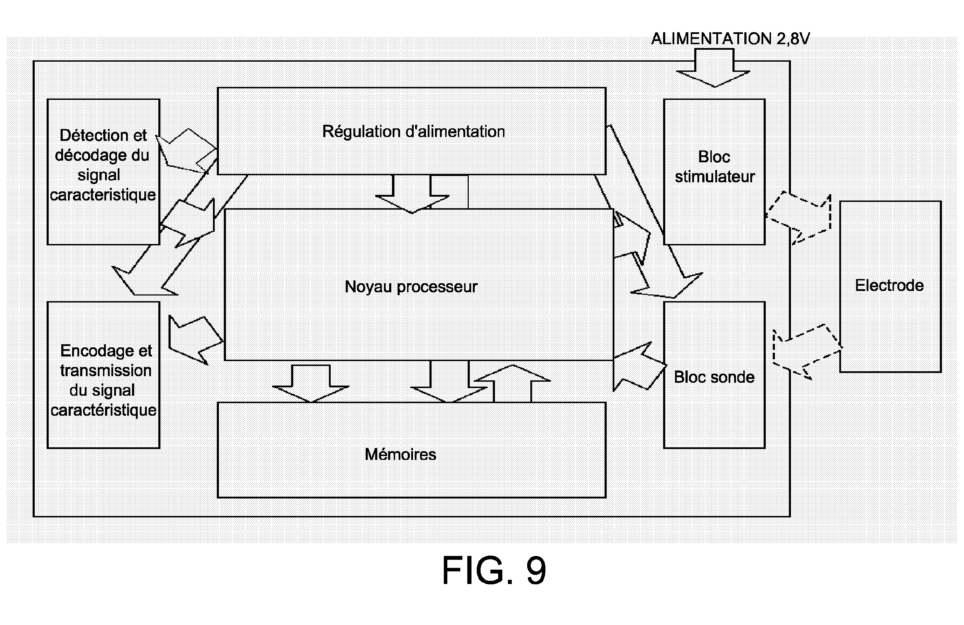

Le module d'électrode représenté de face dans la figure 5 est représenté vu de dessus selon la coupe A-A dans la figure 6. On a représenté pour plus de clarté les pistes conductrice du circuit imprimé de la face supérieure 24a et de la face inférieure 24b, le circuit imprimé 22 ainsi que quatre connecteurs 27 permettant de relier entre eux les modules d'électrodes par l'intermédiaire des fils conducteurs coaxiaux des mailles du filet et de les relier aux pistes conductrices 24a,24b du circuit imprimé. Les pistes conductrices permettent de connecter l'ASIC contenant la régulation d'alimentation, le condensateur pour stimuler et/ou le bloc sonde (voir figure 9) à l'électrode. L'ASCII est relié aux pistes d'alimentation du module. Une régulation d'énergie permet d'avoir le bon niveau de tension d'alimentation des divers blocs (noyau processeur, mémoires, sonde, stimulateur, détection et décodage du signal caractéristique...). Il permet la charge du condensateur servant à la stimulation.

Le bloc de stimulateur permet de délivrer l'impulsion électrique pour stimuler la zone du coeur sur laquelle le module est implanté. Ce stimulus est déclenché par le noyau processeur par la commande de l'interrupteur (voir figure 3).

Le bloc sonde permet d'apporter l'information sur l'état du coeur (dépolarisation repolarisation, ou pas d'activité électrique : tension constante) sur la zone sur laquelle le module est implanté. Cette information est transmise au noyau processeur.

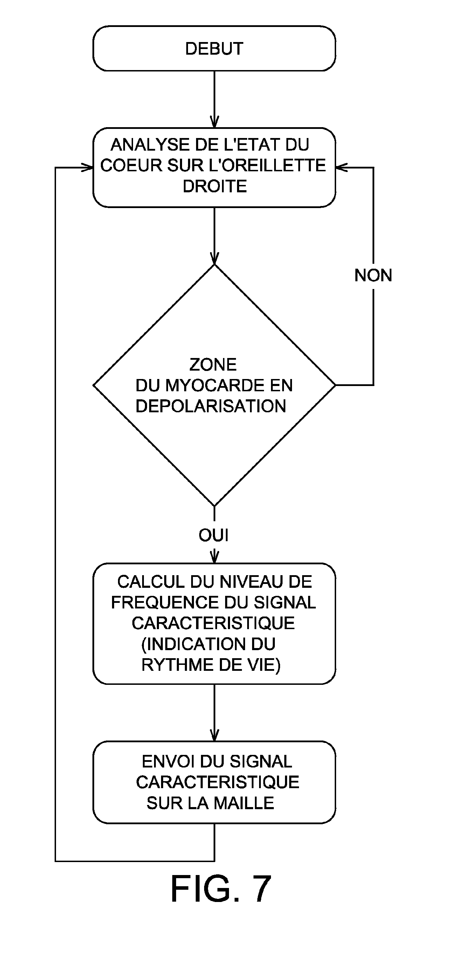

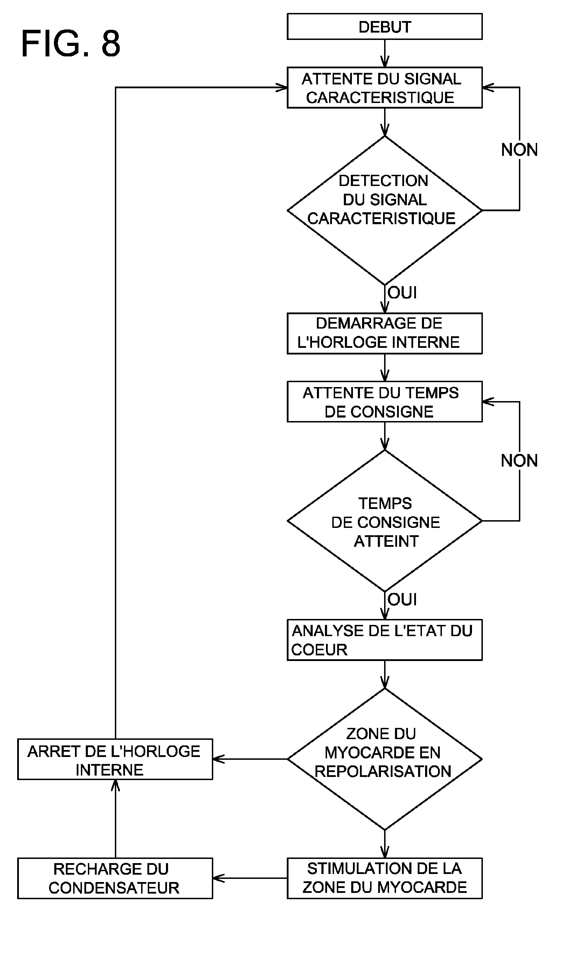

Le noyau processeur permet de commander et de communiquer avec toutes les fonctions du module (stimulation, sonde, décodage, mémoires...) selon le logiciel embarqué qui tient compte des organigrammes (figure 7 et 8). Ce logiciel est stocké sur le bloc mémoires.

Le fonctionnement à proprement dit des différents modules d'électrodes va être maintenant étudié plus en détail.

Dans la figure 2, le module d'électrode 13 situé sur l'oreillette droite est configuré en mode sonde. Il permet d'obtenir l'asservissement des modules d'électrodes en mode de stimulation 14a, 14b reliés par le filet en détectant l'onde de dépolarisation de l'oreillette droite et en synchronisant les stimulations. Comme l'oreillette droite fonctionne normalement, le module d'électrode 13 en mode sonde perçoit les battements cardiaques normaux du patient. Il détermine le rythme cardiaque en calculant le temps écoulé entre deux dépolarisations successives et il transmet cette information aux modules d'électrodes configurés en mode stimulation. Par défaut, le rythme du stimulateur est le rythme de repos, donc le signal caractéristique sera codé en fréquence par défaut à 1 kHz.

Lorsque le module d'électrode en mode sonde détecte la dépolarisation de la zone du myocarde où il est implanté, il émet, par l'intermédiaire des mailles du filet, un signal caractéristique en tension (voir figure 4) aux autres modules d'électrodes positionnés sur les ventricules, en codant ledit signal en fréquence de 1 kHz, 10 kHz et 100 kHz, selon le rythme cardiaque calculé. Il informe ainsi les autres modules d'électrodes du début d'un nouveau cycle coordonné de dépolarisation, contraction et repolarisation du myocarde, à une fréquence donnée. Toutes ces étapes réalisées par le module d'électrode en mode sonde sont schématisées dans la figure 7. Le module d'électrode en mode sonde analyse l'état du coeur en captant les signaux électriques envoyés par le coeur. Il calcule alors le niveau de fréquence du signal électrique reçu si la zone du myocarde sur laquelle il est positionné est en état de dépolarisation, et il envoie ce signal électrique après l'avoir codé en fréquence aux modules d'électrodes en mode stimulation situés sur le filet.

Les modules d'électrodes situés sur les ventricules sont configurés en mode stimulateur. Leurs étapes de fonctionnement en mode de stimulation sont illustrées dans la figure 8. Comme on peut le voir dans cette figure, les modules d'électrodes en mode de stimulation sont en attente du signal caractéristique du début de cycle de dépolarisation codé en fréquence. L'arrivée de ce signal dans un module d'électrode déclenche un compteur ou une horloge interne. Chaque module d'électrode est programmé avec un temps de consigne qui lui est propre. Ce temps de consigne est déterminé à partir de la position du module d'électrode sur le myocarde et du codage en fréquence (c'est-à-dire du rythme de vie du patient équipé) du signal caractéristique. Cette information sur le rythme de vie est fournie par le module d'électrode en mode sonde positionné sur l'oreillette droite. Comme on l'a vu précédemment, la fréquence cardiaque est codée, selon 3 niveaux, sur la fréquence du signal caractéristique pour fournir le rythme de vie du patient : rythme de repos, actif ou « super actif ». Pour chaque rythme de vie et pour une position donnée du module d'électrode en mode stimulateur sur les ventricules, il existe un temps de consigne déterminé. Une fois le temps de consigne atteint, le module d'électrode analyse l'état du coeur. Si la zone du myocarde où est situé le module d'électrode est en repolarisation, alors l'horloge interne est arrêtée et le module d'électrode retourne dans un état d'attente d'un signal caractéristique en provenance du module d'électrode en mode sonde. Si la zone du myocarde n'est pas état de repolarisation à la fin du temps de consigne, alors le module d'électrode déclenche une stimulation de la zone du myocarde concernée, le condensateur est rechargé et l'horloge interne est arrêtée. Ainsi, pour chaque rythme de vie, nous coordonnons la dépolarisation du myocarde des ventricules, en vue de les resynchroniser et ainsi pallier à la pathologie du patient.

Chaque module d'électrode peut avoir une fonction déterminée de sonde ou de stimulateur, mais il peut également être équipé à la fois d'une électronique de traitement et d'une électronique de commande de manière à pouvoir exercer ces deux fonctions selon le besoin. Dans ce cas, le module d'électrode sera configuré selon le cas en mode sonde ou stimulation. Pour configurer à tout instant et à distance les modules d'électrodes, on peut disposer dans le boîtier de commande 10 un moyen de communication permettant de configurer par télétransmission chacun des modules d'électrodes du stimulateur.

Dans l'exemple décrit plus haut, les modules d'électrodes situés sur les ventricules et celui situé sur l'oreillette droite sont constitués des mêmes éléments matériels. Les modules d'électrodes sont en effet constitués d'une électrode et d'une électronique de traitement et de commande. Ils permettent de traiter tous signaux provenant du coeur ou de la maille et d'exercer une commande sur le déclenchement d'une stimulation. Les modules d'électrodes positionnés sur les ventricules observent, par l'électrode qui les équipe, la zone sur laquelle ils sont positionnés. Quand le compteur atteint le temps de consigne pour un de ces modules d'électrodes, il n'envoie une stimulation que si la zone du coeur sur laquelle il est positionné n'est pas en état de repolarisation. Dans ce cas, il stimule cette zone par l'intermédiaire de son électrode en y envoyant une décharge électrique par exemple à l'aide d'un condensateur. Pour engendrer une contraction, la stimulation des cellules du myocarde doit être supérieure à un seuil de stimulation connue grâce à la courbe de Lapicque. La stimulation correspond à une valeur d'environ 2 volts pendant une durée maximale d'une demi-milliseconde. Cette stimulation provoque une dépolarisation des cellules du myocarde dans la zone où se situe l'électrode. Une fois que la stimulation a eu lieu, le condensateur est rechargé et le compteur de l'horloge interne est remis à zéro dans l'attente d'un nouveau signal caractéristique.

Dans cet exemple, le filet reliant entre eux les modules d'électrodes a un rôle de maintien mécanique des électrodes sur le coeur, de communication entre les électrodes et de distribution de l'énergie pour les électrodes. Le filet permet ainsi un fonctionnement coordonné et efficace des modules d'électrodes.

Avantageusement, on préfère, quand il y a plusieurs modules d'électrodes, que le filet permette la distribution d'énergie. Cela évite d'avoir à relier chaque module d'électrode au boîtier de commande par un câble d'alimentation et supprime ainsi une cause d'encombrement.

Dans cet exemple, le stimulateur cardiaque selon l'invention permet de resynchroniser les contractions entre les oreillettes et les ventricules, mais il pourrait également resynchroniser les oreillettes entre elles ou les ventricules entre eux. Il peut également permettre de dynamiser la contraction d'une ou des deux oreillettes, ou/et d'un ou des deux ventricules. La présence du filet équipé de modules d'électrodes permet de stimuler le coeur au plus juste en énergie électrique, au bon moment et au bon endroit. Le filet sert ici à relier entre eux les modules d'électrodes, à réaliser un maintien mécanique des modules d'électrodes sur le coeur, à permettre la communication entre les modules d'électrodes et à la distribution de l'énergie pour les électrodes. Le filet leur confère un fonctionnement coordonné et efficace.

Le stimulateur cardiaque selon l'invention comporte de nombreux avantages.

Le fait de délocaliser au moins une partie de l'électronique contenue dans le boîtier d'un stimulateur cardiaque de l'art antérieur directement sur le coeur permet de libérer de la place pour faire des boîtiers plus petits ou de mettre une pile de plus grande capacité. Ce qui permet d'augmenter la durée d'utilisation des piles et donc d'augmenter la période entre deux changements de piles. De plus, le stimulateur selon l'invention a un encombrement égale ou inférieur aux stimulateurs de l'art antérieur. Les patients qui souffrent d'insuffisance cardiaque pourront donc en être équipés sans problème. Il pourra se substituer au stimulateur cardiaque traditionnel dans la plupart des cas.

Actuellement, la configuration d'un stimulateur cardiaque est telle que le câble d'électrode lui-même n'est qu'un simple conducteur reliant l'électrode et le boîtier de commande, le système d'alimentation (pile) et l'électronique de commande étant contenus dans le boîtier de commande que l'on change régulièrement (environ tous les 4 ans) à cause de l'usure de la pile. Avec une telle configuration, un câble d'électrode correspond à un point de stimulation dans le cas où le câble rejoint une seule électrode, ou plusieurs points de stimulation stimulés au même temps t si le câble est ramifié à une de ses extrémités et relie plusieurs électrodes. Le câble d'électrode peut également relier une électrode (sonde) servant de point d'observation ou d'écoute des signaux électriques parcourant le coeur en un point précis et permettant de coordonner les stimulations réalisées par l'intermédiaire d'une électrode ou de plusieurs électrodes spécifiques de stimulation. En déportant vers le coeur au point de stimulation lui-même tout ou une partie de l'électronique embarquée actuellement présente dans le boîtier du stimulateur, on lève la limite anatomophysiologique due à l'augmentation du nombre de stimulation, ou, d'alimentation pour un pacemaker et de positionnement avec des électrodes ramifiées.

En miniaturisant l'électronique qui est située sur le coeur ou sur la maille s'il y en a une, on réduit la consommation électrique et on augmente la puissance de calcul ou de traitement de l'électronique. Pour miniaturiser l'électronique, on peut utiliser les derniers développements de la microtechnologie (ASIC, FPGA, MENS, ...). La miniaturisation peut consister en la conception d'un système sur puce (SoC : system on chip) à l'aide de la microtechnologie, voire dans le futur avec la nanotechnologie.

De plus, en miniaturisant les électrodes, on réduit leur résistance électrique par rapport à celle des stimulateurs cardiaques de l'art antérieur (de plus de 30 cm de long). On obtient alors une consommation électrique et une résistance électrique de ces électrodes réduites, pour une énergie et une puissance électrique de stimulation délivrée identique. La réduction de la consommation électrique du stimulateur a pour effet d'allonger la durée d'utilisation de la pile.

Un des avantages de l'utilisation d'un moyen permettant de relier les modules d'électrodes entre eux, par exemple un filet, est qu'on peut asservir l'ensemble des modules d'électrodes situés sur ce filet et conditionner leur fonctionnement au résultat d'un autre module d'électrode situé en dehors du filet et servant de sonde.

De plus, en utilisant un filet, on n'a pas besoin de placer précisément les électrodes sur le coeur. Les modules d'électrodes peuvent être placés sur une partie ou la totalité du coeur selon un schéma régulier ou non.

Un autre avantage du filet est qu'il peut aussi servir de filet de contention contre l'hypertrophie des ventricules due à une insuffisance cardiaque.

Par ailleurs, des problèmes de robustesse peuvent se poser avec la technologie des stimulateurs cardiaques actuels. La protection de ces systèmes contre les sources d'agression ou de brouillage externes comme les champs électromagnétiques ou les champs de particules est très difficile et rarement assurée par les fabricants. Avec les technologies actuelles, si une électrode, le boîtier de commande, ou la pile deviennent défaillant, c'est tout le dispositif du stimulateur cardiaque qui devient défaillant, entraînant de graves conséquences pour le patient. Dans le stimulateur cardiaque selon l'invention, les modules d'électrodes peuvent fonctionner de manière indépendante. Si un module d'électrode est endommagé, les autres modules d'électrodes peuvent continuer à fonctionner.

Le stimulateur cardiaque selon l'invention rend possible la stimulation de nombreux sites du coeur en péricardique ou en endocavitaire. En effet, ce stimulateur rend possible d'avoir un nombre de points de stimulation beaucoup plus important que ce qui est effectué par les stimulateurs cardiaques actuels qui sont limités à n'utiliser que trois câbles d'électrodes au maximum à cause de leur encombrement.

Avec notre invention, nous augmentons la robustesse et la sûreté de fonctionnement du stimulateur cardiaque. En effet, en disposant des électrodes miniatures sur un maillage, nous ne sommes plus limités en nombre d'électrodes pour des raisons anatomophysiologiques ou de résistance électrique de câbles d'électrodes ramifiés difficiles à alimenter avec les technologies de pile usuelle. Nous pouvons alors avoir un nombre très important de points de stimulation, plus important que ce que la technologie des stimulateurs actuels ne permet. En ayant un grand nombre de points de stimulation, on peut par ailleurs assurer une redondance des points de stimulation et des voies d'alimentation grâce à la présence du filet. Cette redondance de points de stimulation permet entres autres de pallier à la démarche pragmatique du patricien qui cherche lors de l'opération chirurgicale à bien positionner les électrodes.

### BIBLIOGRAPHIE

**[1]** US 2000/6067470 A1 ; « System and method for multiple site biphasic stimulation to revert ventricular arrhythmias »
**[2]** US 2002/0082647 A1 ; « Cardiac disease treatment and device ».

## Revendications

1. Stimulateur cardiaque implantable comprenant au moins un câble conducteur (11) reliant électriquement un boîtier de commande (10) à au moins une électrode (18) placée en un point du coeur d'un patient, le boîtier de commande (10) comprenant un système d'alimentation et un système électronique, **caractérisé en ce qu'**au moins une partie du système électronique est délocalisée du boîtier de commande (10) et est située au niveau de la au moins une électrode (18) pour former un module d'électrode (13,14a,14b,15,21), le module d'électrode étant placé à la surface du coeur.

2. Stimulateur cardiaque implantable selon la revendication 1, **caractérisé en ce qu'**au moins une partie du système d'alimentation est délocalisée du boîtier de commande (10) et est située au niveau d'au moins un module d'électrode (13,14a,14b,15,21).

3. Stimulateur cardiaque implantable selon la revendication 1, **caractérisé en ce que** la partie du système électronique délocalisée est une électronique de traitement et/ou de commande, cette électronique permettant au module d'électrode (13,14a,14b,15,21) de sonder et/ou de stimuler le coeur.

4. Stimulateur cardiaque implantable selon la revendication précédente, **caractérisé en ce que** l'électronique de traitement permettant au module d'électrode (13,21) de sonder le coeur comprend au moins un capteur.

5. Stimulateur cardiaque implantable selon l'une quelconque des revendications 3 ou 4, **caractérisé en ce que** l'électronique de traitement permettant au module d'électrode (13,21) de sonder le coeur comprend un moyen apte à sélectionner des signaux de fréquence déterminée.

6. Stimulateur cardiaque implantable selon la revendication précédente, **caractérisé en ce que** le moyen apte à sélectionner des signaux de fréquence déterminée est un filtre.

7. Stimulateur cardiaque implantable selon la revendication 3, **caractérisé en ce que** l'électronique de commande permettant au module d'électrode (14a,14b,15,21) de stimuler le coeur comprend un moyen apte à délivrer une stimulation électrique au coeur.

8. Stimulateur cardiaque implantable selon la revendication précédente, **caractérisé en ce que** ledit moyen apte à délivrer une stimulation électrique au coeur est un condensateur (17).

9. Stimulateur cardiaque implantable selon l'une quelconque des revendications 1 ou 3, **caractérisé en ce que** la partie du système électronique délocalisée est un système sur puce.

10. Stimulateur cardiaque implantable selon l'une quelconque des revendications précédentes, **caractérisé en ce qu'**il comprend au moins deux modules d'électrodes (14a,14b) reliés entre eux par un moyen qui entoure au moins une partie du coeur.

11. Stimulateur cardiaque implantable selon la revendication précédente, **caractérisé en ce que** le moyen qui entoure le coeur sert au maintien mécanique desdits au moins deux modules d'électrodes (14a,14b) sur la au moins une partie du coeur.

12. Stimulateur cardiaque implantable selon l'une quelconque des revendications 10 ou 11, **caractérisé en ce que** le moyen est un moyen servant à la contention de la partie du coeur entourée par ledit moyen.

13. Stimulateur cardiaque implantable selon l'une quelconque des revendications 10 à 12, **caractérisé en ce que** le moyen est un moyen servant à la communication électrique entre les modules d'électrodes (14a,14b).

14. Stimulateur cardiaque implantable selon l'une quelconque des revendications 10 à 13, **caractérisé en ce que** ledit moyen est un moyen servant à la distribution d'énergie entre les modules d'électrodes (14a,14b).

15. Stimulateur cardiaque implantable selon la revendication 14, **caractérisé en ce que** ledit moyen ou un module d'électrode (14a,14b) situé sur ledit moyen est relié au boîtier de commande (10) par un câble conducteur.

16. Stimulateur cardiaque implantable selon l'une quelconque des revendications 13 ou 14, **caractérisé en ce que** ledit moyen est un câble coaxial multifilaire.

17. Stimulateur cardiaque implantable selon l'une quelconque des revendications 10 à 14, **caractérisé en ce que** le moyen est un filet (12) comportant un maillage.

18. Stimulateur cardiaque implantable selon la revendication 17, **caractérisé en ce que** le maillage du filet (12) est réalisé à partir de conducteurs hélicoïdaux multifilaires et coaxiaux, recouverts d'un matériau électriquement isolant et biocompatible avec les tissus humains avec lesquels ils sont en contact.

19. Stimulateur cardiaque implantable selon l'une quelconque des revendications précédentes, **caractérisé en ce que** le système électronique comprend en outre un moyen de communication permettant de configurer à distance les modules d'électrodes (13,14a,14b,15,21), le moyen de communication étant situé dans le boîtier de commande (10) et comportant une antenne fonctionnant par télétransmission.

20. Stimulateur cardiaque implantable selon la revendication précédente, **caractérisé en ce que** seule l'antenne du moyen de communication est situé dans le boîtier de commande (10), le reste des éléments constituant le moyen de communication étant localisé sur le moyen permettant de relier les modules d'électrodes (14a,14b).

21. Stimulateur cardiaque implantable selon l'une quelconque des revendications 3 à 20, **caractérisé en ce qu'**au moins un module d'électrode (13) est configuré en mode sonde et transmet des informations sur l'état du coeur aux autres modules d'électrodes (14a, 14b, 15).

22. Stimulateur cardiaque implantable selon l'une quelconque des revendications 3 à 20, **caractérisé en ce qu'**au moins un module d'électrode est configuré en mode sonde et en mode stimulation.

23. Stimulateur cardiaque implantable selon l'une quelconque des revendications 10 à 22, **caractérisé en ce que** tous les modules d'électrodes (13,14a,14b,15,21) sont situés sur le moyen permettant de relier entre eux les modules d'électrodes.

24. Stimulateur cardiaque implantable selon la revendication 21, **caractérisé en ce qu'**au moins un module d'électrode (13) configuré en mode sonde n'est pas situé sur le moyen permettant de relier entre eux des modules d'électrodes, mais lui est relié électriquement.

25. Stimulateur cardiaque implantable selon les revendications 15 et 21 prises ensemble, **caractérisé en ce qu'**il comprend un module d'électrode (13) configuré en mode sonde apte à exécuter la suite d'étapes suivantes :
a)- analyser l'état électrique du coeur au niveau où le module d'électrode (13) configuré en mode sonde est situé,
b)- à la réception d'un signal électrique provenant du coeur, calculer le niveau de fréquence dudit signal pour déterminer si le coeur est en état de dépolarisation ou en état de repolarisation,
c)- si le coeur est en état de dépolarisation, alors envoyer un signal codé en fréquence sur le moyen permettant l'échange d'information entre les modules d'électrodes (14a,14b,15,21), le codage en fréquence étant indicatif du temps séparant deux états de repolarisation du coeur.

26. Stimulateur cardiaque implantable selon la revendication précédente, **caractérisé en ce qu'**il comprend au moins un module d'électrode (14a,14b,15) configuré en mode stimulation qui est apte à exécuter la suite d'étapes suivantes :
a)- attendre un signal codé en fréquence provenant d'un module d'électrode (13) en mode sonde,
b)- après la détection du signal codé, démarrer l'horloge interne et attendre pendant un temps de consigne déterminé,
c)- analyser l'état électrique du coeur au niveau où le module d'électrode (14a,14b,15) en mode stimulation est situé,
d)- si il y a réception par le module d'électrode (14a,14b,15) configuré en mode stimulation d'un signal électrique en provenance du coeur dont la valeur est supérieure à un seuil déterminé, alors l'horloge interne est arrêtée et le temps de consigne est remis à zéro,
e)- si aucun signal électrique provenant du coeur n'est reçu par le module d'électrode (14a,14b,15) en mode stimulation, alors ledit module d'électrode délivre une stimulation électrique au niveau du point du coeur où il est situé, l'horloge interne est arrêtée et le temps de consigne est remis à zéro.
